# EUROPEAN PATENT APPLICATION

(11) **EP 4 733 761 A1**
(43) Date of publication of application: **29.04.2026**
(21) Application number: 24825937.6
(22) Date of filing: 19.06.2024
(51) Int. Cl.: G01N 33/50, C07K 14/00, G01N 33/15

(54) **METHOD FOR SCREENING FOR PROTEINS ASSOCIATED WITH NEURODEGENERATIVE DISEASES AND THERAPEUTIC OR PROPHYLACTIC DRUGS FOR NEURODEGENERATIVE DISEASES**

(30) Priority: 22.06.2023 JP 2023102490
(71) Applicant: National Institute of Advanced Industrial Science and Technology, Chiyoda-ku Tokyo 100-8921 (JP)
(72) Inventor: TOMITA, Shunsuke, Tsukuba-shi, Ibaraki 305-8560 (JP); ISHIHARA, Sayaka, Tsukuba-shi, Ibaraki 305-8560 (JP)
(74) Representative: HGF
(86) International application number: PCT/JP2024/022222
(87) International publication number: WO 2024/262536

(57) **Abstract**

A simple and rapid method is provided for screening proteins associated with neurodegenerative diseases or compounds useful for the treatment or prevention of neurodegenerative diseases. The method uses a probe comprising a polypeptide comprising a dipeptide repeat sequence consisting of repeating units selected from the group consisting of proline-arginine, glycine-arginine, proline-alanine, glycine-alanine, and glycine-proline, and an environment-sensitive fluorophore covalently linked thereto.

## Description

### TECHNICAL FIELD

The present invention relates to methods for screening proteins associated with neurodegenerative diseases and to methods for screening therapeutic or prophylactic agents for the treatment of neurodegenerative diseases.

### BACKGROUND

As the elderly population is currently increasing, the number of patients with neurodegenerative diseases is on the rise, thus making the development of treatment methods for these diseases an urgent need. Neurodegenerative diseases include dementia, such as Alzheimer's disease (AD) and frontotemporal lobar degeneration (FTLD), as well as amyotrophic lateral sclerosis (ALS) and Parkinson's disease (PD). A common pathological feature of neurodegenerative diseases is the increased accumulation and deposition of abnormal proteins called inclusion bodies. For example, in ALS and FTLD, which belong to the same disease spectrum as ALS, the abnormal aggregation of RNA-binding proteins, such as fused in sarcoma (FUS) and TAR DNA-binding protein 43 kDa (TDP-43), is observed inside and outside of neurons. In addition, it has recently been shown that a physical phenomenon called "liquid-liquid phase separation (LLPS)" is associated with the aggregation process of these proteins, and it has been suggested that the dysfunction of LLPS regulators, such as karyopherin β2 (Kapβ2), is associated with the pathology of neurodegenerative diseases (Non-patent Document 1).

On the other hand, it has also been reported that the abnormal expansion of a hexanucleotide (GGGGCC) repeat in intron 1 of the chromosome 9 open reading frame 72 (*C9orf72*) gene may contribute to the pathogenesis of ALS and FTLD. There are several hypotheses regarding the aberrant expansion of repeats and the pathogenesis of ALS/FTLD, one of which is the expression of neurotoxic peptides via initiation codon-independent translation (repeat-associated non-ATG (RAN) translation). The neurotoxic peptides expressed from the hexanucleotide repeat sequences by RAN translation are called dipeptide repeat proteins (DPRs), which consist of proline-arginine (PR), glycine-arginine (GR), proline-alanine (PA), glycine-alanine (GA), or glycine-proline (GP) repeats. A model has been reported, which suggests that arginine-rich DPRs (i.e., polyPRs and polyGRs) may disrupt LLPS by inhibiting the interaction between FUS and Kapβ2 (Non-patent Document 2).

Based on these findings, it is hoped that the elucidation of the interaction between proteins and DPRs will lead to an understanding of the pathology of neurodegenerative diseases, such as ALS and FTLD, and ultimately to the development of treatment methods for neurodegenerative diseases. However, conventional common methods for analyzing protein-protein interactions, such as co-immunoprecipitation, nuclear magnetic resonance (NMR), X-ray crystallography, and ultracentrifugal analysis, are labor-intensive, time-consuming, and expensive, thus making their use impractical to comprehensively screen for proteins that interact with DPRs.

### REFERENCE DOCUMENT LIST

### NON-PATENT DOCUMENTS

Non-patent Document 1: Acta Neuropathol., 2016; 132(2):159-173
Non-patent Document 2: Nat. Commun., 2021; 12(1):5301

### SUMMARY OF THE INVENTION

### PROBLEM TO BE SOLVED BY THE INVENTION

The purpose of the present invention is to provide a method for easily and rapidly screening for proteins associated with neurodegenerative diseases, or compounds useful for the treatment or prevention of neurodegenerative diseases, based on the interaction between DPRs and proteins.

### MEANS FOR SOLVING THE PROBLEM

After extensive research, the inventors discovered that proteins that interact with DPRs and compounds that inhibit the interaction between DPRs and proteins can be detected using a probe comprising a polypeptide comprising a dipeptide repeat sequence and an environment-sensitive fluorophore covalently linked thereto.

The present invention, according to a first embodiment, is a method of screening for a protein associated with a neurodegenerative disease, which includes the following steps: (1) contacting a probe comprising a polypeptide comprising a dipeptide repeat sequence and an environment-sensitive fluorophore covalently linked thereto, with a sample containing a protein, wherein the dipeptide repeat sequence consists of a repeating unit selected from the group consisting of proline-arginine, glycine-arginine, proline-alanine, glycine-alanine, and glycine-proline; and (2) measuring the fluorescence intensity of the probe.

In addition, according to one embodiment, the present invention provides a method of screening an agent for the treatment or prevention of a neurodegenerative disease, which comprises the following steps: (1) contacting, in the presence of a candidate compound, a probe comprising a polypeptide comprising a dipeptide repeat sequence and an environment-sensitive fluorophore covalently linked thereto, with an LLPS-associated protein or a nuclear pore complex family protein, wherein the dipeptide repeat sequence consists of a repeating unit selected from the group consisting of proline-arginine, glycine-arginine, proline-alanine, glycine-alanine, and glycine-proline; and (2) measuring the fluorescence intensity of the probe.

The sample is preferably a biological sample.

The dipeptide repeat sequence preferably comprises 3 to 200 of the repeating units.

The environment-sensitive fluorophore is preferably selected from a group consisting of fluorophores with a naphthalenesulfonic acid core, fluorophores with a benzofurazan core, fluorophores with a xanthene core, fluorophores with a pyrene core, and aggregation-induced emission (AIE) fluorophores.

The LLPS-associated protein is preferably FUS.

The nuclear pore complex family protein is preferably karyopherin β2.

The neurodegenerative disease is preferably amyotrophic lateral sclerosis or frontotemporal lobar degeneration.

### EFFECTS OF THE INVENTION

According to the method of the present invention, proteins that interact with DPRs, or compounds that inhibit the interaction between DPRs and proteins, can be detected simply by monitoring the changes in fluorescence intensity of a probe comprising a polypeptide comprising a dipeptide repeat sequence and an environment-sensitive fluorophore covalently linked thereto. Therefore, the method of the present invention enables the simple and rapid screening of proteins that interact with DPRs, or compounds that inhibit the interaction between DPRs and proteins, and is useful for drug discovery for neurodegenerative diseases.

### BRIEF DESCRIPTION OF THE DRAWINGS

[FIG. 1] Figure 1 is a schematic diagram of the screening method for proteins that interact with DPRs.
[FIG. 2] Figure 2 is a schematic diagram of the screening method for compounds that inhibit the interaction between DPRs and proteins.
[FIG. 3] Figure 3 presents the structural formulae of Dnc-PR20 (probe 1) and Dnc-GP20 (probe 2).
[FIG. 4] Figure 4 shows changes in the fluorescence spectrum and intensity of Dnc-PR20 (probe 1) and Dnc-GP20 (probe 2) contacted with Kapβ2.
[FIG. 5] Figure 5 shows changes in the fluorescence spectrum and intensity of Dnc-PR10 (probe 3), Dnc-GP10 (probe 4), Dnc-GA10 (probe 5), Dnc-GR10 (probe 6), and Dnc-PA10 (probe 7) contacted with Kapβ2.
[FIG. 6] Figure 6 shows the changes in fluorescence spectrum and fluorescence intensity of Dnc-PR20 (probe 1) and Dnc-GP20 (probe 2) contacted with FUS.
[FIG. 7] Figure 7 shows changes in the fluorescence spectrum of Dnc-PR20 (probe 1) and Dnc-GP20 (probe 2) contacted with various proteins.
[FIG. 8] Figure 8 is a graph presenting the changes in fluorescence intensity of Dnc-PR20 (probe 1) and Dnc-GP20 (probe 2) contacted with various proteins.
[FIG. 9] Figure 9 shows changes in the fluorescence spectrum and intensity of the Dnc-PR20 (probe 1)/Kapβ2 mixture solution with added polyK50 or IgG.
[FIG. 10] Figure 10 is a graph presenting the changes in fluorescence intensity of the Dnc-PR20 (probe 1)/Kapβ2 mixture solution comprising various additives.
[FIG. 11] Figure 11 is a graph presenting the changes in fluorescence intensity of the Dnc-PR20 (probe 1)/Kapβ2 mixture solution with various protein-protein interaction (PPI) inhibitors added.
[FIG. 12] Figure 12 shows changes in the fluorescence spectrum and intensity of the Dnc-PR20 (probe 1)/Kapβ2 mixture solution with the inhibitor, Compound No. 8, added.

### MODE FOR CARRYING OUT THE INVENTION

The present invention is described in detail below; however, the scope of the present invention is not limited to the embodiments described herein.

According to a first embodiment of the present invention, a method of screening for a protein associated with neurodegenerative diseases, which includes the following steps: (1) contacting a probe comprising a polypeptide comprising a dipeptide repeat sequence and an environment-sensitive fluorophore covalently linked thereto, with a sample containing a protein, wherein the dipeptide repeat sequence consists of a repeating unit selected from the group consisting of proline-arginine, glycine-arginine, proline-alanine, glycine-alanine, and glycine-proline; and (2) measuring the fluorescence intensity of the probe.

First, the probe used in the method of this embodiment is described. In this embodiment, the probe consists of a polypeptide comprising a dipeptide repeat sequence and an environment-sensitive fluorophore covalently linked thereto.

Dipeptide repeat proteins (hereinafter referred to as "DPRs") are neurotoxic proteins associated with neurodegenerative diseases. DPRs have been shown to be produced due to the abnormal amplification of a hexanucleotide (GGGGCC) repeat in intron 1 of the *C9orf72* gene. DPRs are translated from six reading frames in the sense or antisense direction of hexanucleotide repeats and thus consist of two amino acid repeating units of proline-arginine (PR), glycine-arginine (GR), proline-alanine (PA), glycine-alanine (GA), or glycine-proline (GP).

The polypeptide used in the probe in this embodiment comprises a dipeptide repeat sequence comprising any of the above repeating units, i.e., a dipeptide repeat sequence comprising a repeating unit selected from a group consisting of PR, GR, PA, GA and GP. The number of repeating units in a dipeptide repeat sequence is not particularly limited and may be, for example, 3 to 200, 5 to 100, or 5 to 50, but is preferably 5 to 30. In other words, the dipeptide repeat sequence in this embodiment is selected from a group consisting of (PR)ₙ, (GR)ₙ, (PA)ₙ, (GA)ₙ, and (GP)ₙ, where n may be, for example, 3 to 200, 5 to 100, or 5 to 100, but is preferably 5 to 30.

In this embodiment, the polypeptide comprising a dipeptide repeat sequence may consist solely of the dipeptide repeat sequence, or may be have a tag, such as GST, His6, MBP, HA, or FLAG, or an amino acid residue (e.g., lysine residue) that is suitable for modification with an environment-sensitive fluorophore, added to the N- and/or C-terminus, either directly or via a linker.

The environment-sensitive fluorophore used for the probe in this embodiment is not particularly limited and may be any fluorophore having fluorescent properties that change depending on the surrounding environment of the fluorescent molecule. Examples of such fluorophores include those having fluorescence properties that change with the polarity, pH, or degree of crowding that surrounds the fluorescent molecule.

Examples of fluorophores having fluorescence properties that change depending on the polarity around the fluorescent molecule are fluorophores that have a naphthalenesulfonic acid core, such as 5-dimethylaminonaphthalene-1-sulfonyl (dansyl), 1-anilinonaphthalene-8-sulfonic acid (ANS), N-methyl-2-anilinonaphthalene-6-sulfonic acid (MANS), and 2-p-toluidinylnaphthalene-6-sulfonic acid (TNS); fluorophores that have a benzofurazan core, such as 4-(N,N-dimethylaminosulfonyl)-2,1,3-benzoxadiazole (DBD), 7-nitro-2,1,3-benzoxadiazole (NBD), 4-(aminosulfonyl)-2,1,3-benzoxadiazole (ABD), and ammonium 2,1,3-benzoxadiazole-4-sulfonate (SBD); and fluorescent derivatives thereof.

Examples of fluorophores having fluorescence properties that change depending on the pH around the fluorescent molecule are fluorophores that have a xanthene core, such as fluorescein, fluorescein isothiocyanate (FITC), 5(6)-carboxyfluorescein (5(6)-FAM), 2'-7'-bis(carboxyethyl)-5(6)-carboxyfluorescein (BCECF), and seminaphtharhodafluorescein (SNARF); fluorophores that have a pyrene core, such as 8-hydroxypyrene-1,3,6-trisulfonic acid trisodium salt (HTPS); and fluorescent derivatives thereof.

Examples of fluorophores having fluorescence properties that change depending on the degree of crowding around the fluorescent molecule are AIE fluorophores, such as tetraphenylethylene (TPE), 10,10',11,11'-tetrahydro-5,5'-bidibenzo[a,d][7] annulenylidene (THBA), and 1,1,2,3,4,5-hexaphenylsilole (HPS), as well as fluorescent derivatives thereof.

A preferred environment-sensitive fluorophore in this embodiment may be dansyl, NBD, DBD, or TPE, or a fluorescent derivative thereof.

The probe in this embodiment consists of a polypeptide comprising a dipeptide repeat sequence to which an environment-sensitive fluorophore is covalently linked. The environment-sensitive fluorophore may be linked to any one or more positions of the polypeptide, such as either or both the N- and C-termini and/or a side chain of an amino acid residue in the polypeptide. In the probe in this embodiment, the environment-sensitive fluorophore is preferably linked to the N- and/or C-terminus of the polypeptide, and the N-terminus is particularly preferred.

The probe in this embodiment can be prepared by synthesizing a polypeptide and labeling it with a fluorophore using a conventionally known method. For example, the probe in this embodiment may be prepared by chemically synthesizing or biosynthesizing a polypeptide comprising a dipeptide repeat sequence and labeling an amino group in the polypeptide with an environment-sensitive fluorophore activated by an active ester group, such as an N-hydroxysuccinimide (NHS) ester group and pentafluorophenyl (PFP) ester group, a maleimide group, an isothiocyanate group, or a halogenated alkyl group.

In the method of this embodiment, the above probe is brought into contact with a sample comprising protein. The sample in this embodiment is not particularly limited as long as it contains protein, and may contain one or multiple types of protein. The type of protein is also not particularly limited and may be any protein that can be expressed in any living organism, such as a vertebrate, preferably a mammal such as a mouse, rat, rabbit, dog, monkey, or human, where a human is particularly preferred, and may include known and unknown proteins. In addition, the protein targeted by the method of this embodiment may be the entire protein or a peptide fragment thereof, and it may also include any post-translational modification.

The sample in this embodiment is preferably a biological sample, such as a biological fluid, an extract from a cell or tissue, or a supernatant of a cell or tissue culture. The biological fluid may be blood, plasma, serum, cerebrospinal fluid (CSF), saliva, or urine. The cell may be a neuron, glial cell, fibroblast, stem cell, endothelial cell, or pericyte, and may be a primary cultured cell derived from a living organism or a cell from a previously established cell line. The tissue may be central nervous tissue, peripheral nervous tissue, or a brain organoid. The biological sample may be derived from any living organism, such as from a vertebrate, preferably from a mammal such as a mouse, rat, rabbit, dog, monkey, or human, where a human is particularly preferred.

To contact the protein-containing sample and the probe, a reaction solution comprising a mixture of the protein-containing sample and probe can be prepared and incubated for a certain period of time. The ionic strength and pH of the reaction solution are not particularly limited, and the reaction solution may comprise any buffer and/or salt and may further comprise an organic solvent, such as ethanol, or glycerin. Examples of buffers are HEPES, MOPS, MES, EPPS, Tris, phosphoric acid, acetic acid, citric acid, glycine, etc. Examples of salts are NaCl, KCl, MgCl₂, Na₂SO₄, K₂SO₄, MgSO₄, NaI, NaSCN, etc.

The reaction solution in this embodiment can preferably conform to *in vivo* conditions. Therefore, the pH of the reaction solution is preferably 4.0 to 8.0, and the ionic strength is preferably 10 to 500 mM. The reaction solution in this embodiment may be a brain tissue extract or a buffer solution prepared according to the composition thereof, e.g., HEPES-buffered artificial cerebrospinal fluid (aCSF).

The final protein concentration in the reaction solution may be 1 nM to 1000 µM, and be preferably 10 nM to 100 µM. If the protein concentration in the sample to be added is unknown, the sample may be used in a step dilution as appropriate. The incubation time of the reaction solution may be in the range of 10 seconds to 72 hours.

The fluorescence intensity of the probe is then measured. In this method, fluorescence intensity can be measured at excitation wavelengths of 300 to 500 nm and fluorescence wavelengths of 400 to 700 nm. In this method, fluorescence intensity may be measured at one or more sets of excitation/fluorescence wavelengths, for example, one, two, three, or four sets selected from excitation (nm)/fluorescence wavelength (nm) of 340/520, 330/480, 345/505, 360/530, etc.

To determine whether the protein in the sample has bound to the probe, a control without the protein (such as a solution comprising only the probe) may be analyzed in parallel and be compared, or the results may be compared with the results of a previously analyzed control. Alternatively, a reaction solution comprising a probe mixed with a sample comprising only proteins that have previously been shown not to interact with DPRs may be used as a control. In this method, if the fluorescence intensity of the probe in contact with the sample is significantly increased compared to the fluorescence intensity of the probe in the control, it can be determined that a protein that can interact with DPRs, i.e., a protein that can be associated with neurodegenerative diseases, is present in the sample.

"Neurodegenerative disease" is a general term for progressive diseases in which neurons in the central nervous system gradually degenerate, leading to cell death. Neurodegenerative diseases in this embodiment include, but are not limited to, frontotemporal lobar degeneration (FTLD), such as frontotemporal dementia (FTD), progressive non-fluent aphasia (PNFA), and semantic dementia (SD); amyotrophic lateral sclerosis (ALS); Alzheimer's disease (AD); Parkinson's disease (PD); multiple sclerosis (MS); Huntington's disease (HD); dementia with Lewy bodies (DLB); corticobasal syndrome (CBS); and progressive supranuclear palsy (PSP). The neurodegenerative disease in this embodiment is preferably ALS or FTLD.

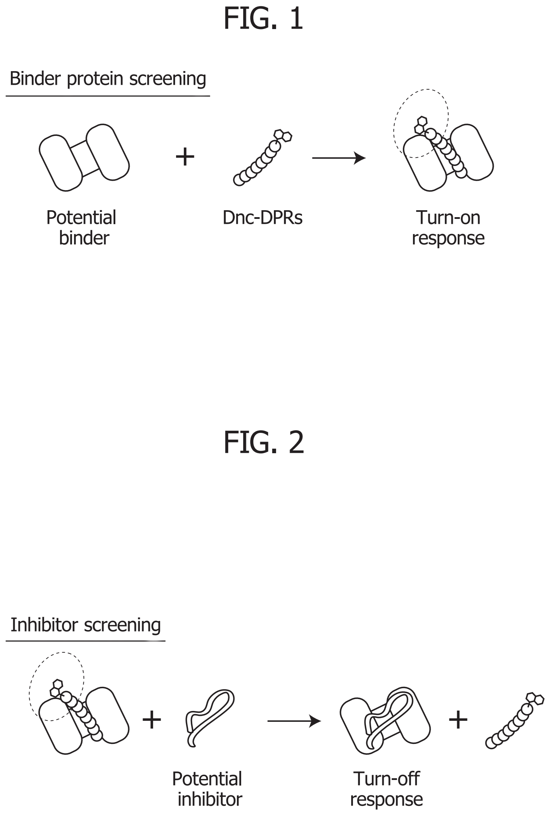

A schematic of the method is shown in Figure 1. In Figure 1, DPRs with dansyl (Dnc-DPRs) are shown as an example of probes. When the probe interacts with a protein that can interact with DPRs ("Potential binder" in the figure), the fluorescence intensity of the probe increases (turn-on response). Thus, the method of this embodiment allows for the screening of proteins that can interact with DPRs, i.e., proteins that can be associated with neurodegenerative diseases, based on the increased fluorescence intensity of the probe.

The present invention, according to a second embodiment, is a method of screening an agent for the treatment or prevention of a neurodegenerative disease, comprises the following steps: (1) contacting, in the presence of a candidate compound, a probe comprising a polypeptide comprising a dipeptide repeat sequence and an environment-sensitive fluorophore covalently linked thereto with a liquid-liquid phase separation-associated protein or a nuclear pore complex family protein, wherein the dipeptide repeat sequence is selected from the group consisting of proline-arginine, glycine-arginine, proline-alanine, glycine-alanine and glycine-proline; and (2) measuring the fluorescence intensity of the probe.

In this embodiment, the terms "dipeptide repeat sequence," "environment-sensitive fluorophore," "probe consisting of a polypeptide comprising a dipeptide repeat sequence and an environment-sensitive fluorophore covalently linked thereto," and "neurodegenerative disease" are as defined in the first embodiment.

The term "treatment" (and the grammatical variants thereof) in this embodiment means to stop or alleviate the progression and deterioration of the pathology of a neurodegenerative disease in a subject, including the complete cure of the disease as well as the alleviation of various symptoms of the disease. The term "prevention" (and the grammatical variants thereof) in this embodiment refers to preventing the development of neurodegenerative diseases in subjects who are at risk of the onset of such diseases. The "subject" may be any mammal, but is preferably a human. Subjects can be any age, including infants, young adults, adolescents, adults, and geriatric subjects.

In this method, a probe comprising a polypeptide comprising a dipeptide repeat sequence and an environment-sensitive fluorophore covalently linked thereto is contacted, in the presence of a candidate compound, with a liquid-liquid phase separation (LLPS)-associated protein or nuclear pore complex (NPC) family protein.

The term "liquid-liquid phase separation (LLPS)-associated protein" refers to proteins associated with the formation of liquid-liquid phase separation (LLPS). LLPS-associated proteins are found in various species, including eukaryotes, prokaryotes, and viruses, but all share a common intrinsically disordered region, such as the low complexity (LC) domain. Information on LLPS-associated proteins can be obtained from numerous databases such as PhaSepDB (http://db.phasep.pro/), DrLLPS (http://llps.biocuckoo.cn/), LLPSDB (http://bio-comp.org.cn/llpsdb/home.html), and PhaSePro (https/phasepro.elte.hu/).

The LLPS-associated protein in this embodiment may preferably be FET family proteins or heteronuclear ribonucleoproteins. FET family proteins are RNA-binding proteins with LC domains on their N-terminal ends. FET family proteins include FUS (fused in sarcoma, also known as TLS (translocated in liposarcoma)), EWSR1 (Ewing sarcoma breakpoint region 1, also known as RNA-binding protein EWS), and TAF15 (TATA-binding protein-related factor 15). Heteronuclear ribonucleoproteins (hnRNPs) are RNA-binding proteins with LC domains on their C-terminal ends. The hnRNPs include, but are not limited to, TDP-43 (TAR DNA binding protein 43 kDa), hnRNP A0, hnRNP A1, hnRNP A2, hnRNP A3, hnRNP H1, hnRNP H2, hnRNP H3, AUF1, and PTBP1. The LLPS-associated protein in this embodiment is preferably FUS.

The NPC family proteins are proteins involved in molecular transport between the cytoplasm and nucleus and regulate LLPS. NPC family proteins include, but are not limited to, karyopherin β2 (also known as importin β2 or transportin 1), karyopherin β2b (also known as importin 3 or transportin 2), karyopherin β1 (also known as importin β1), importin α1, and importin α2. The NPC family protein in this embodiment is preferably karyopherin β2 (Kapβ2).

The LLPS-associated protein and NPC family protein in this embodiment may be derived from any vertebrate, preferably mammals, such as mice, rats, rabbits, dogs, monkeys, and humans, where humans are preferred.

Amino acid sequences of LLPS-associated proteins and NPC family proteins, as well as the nucleic acid sequence information encoding them, can be obtained from the given databases. For example, the amino acid and gene sequence for human Kapβ2 is available under NCBI Gene ID: 3842, and the amino acid and gene sequence for human FUS is available under NCBI Gene ID: 2521.

The LLPS-associated protein and NPC family protein in this embodiment may also include the variants and homologs thereof with equivalent activity. In other words, the LLPS-associated protein in this embodiment can include proteins comprising an amino acid sequence that has more than 80%, preferably more than 90%, and more preferably greater than 95% identity with the amino acid sequences in the database, as long as their capacity to form LLPS is maintained. Also, NPC family proteins in this embodiment can include proteins comprising an amino acid sequence that has more than 80%, preferably more than 90%, and more preferably greater than 95% identity with the amino acid sequences in the database, as long as their capacity to modulate LLPS is maintained. The identity of the amino acid sequence can be calculated using sequence analysis software or programs that are customarily used in the field (e.g., FASTA and BLAST).

The LLPS-associated protein and NPC family protein in this embodiment may also include proteins in which one to several amino acids in the amino acid sequences registered in the database have been substituted, deleted, inserted, and/or added, provided that their LLPS-forming or LLPS-regulating activities are retained. Here, "one to several" means, for example, 1 to 30, preferably 1 to 10, and more preferably 1 to 5.

LLPS-associated protein and NPC family protein in this embodiment may have a tag, such as GST, His6, MBP, HA, and FLAG, added to the N- and/or C-terminus, directly or via a linker.

The LLPS-associated protein and NPC family protein in this embodiment can be prepared using any previously established genetic engineering method. For example, expression vectors comprising nucleic acids encoding LLPS-associated proteins and NPC family proteins can be created and introduced into host cells such as *E. coli* for expression.

To contact the probe and the LLPS-associated protein or NPC family protein in the presence of the candidate compound, a reaction solution in which the probe, LLPS-associated protein or NPC family protein, and candidate compound are mixed can be prepared and incubated for a certain period of time. The protein concentration, ionic strength, and pH of the reaction solution in this embodiment may be the same as defined in the first embodiment.

The "candidate compound" in this embodiment may be a low molecular weight compound, nucleic acid, protein, peptide, antibody, lipid, or mixture thereof (e.g., an extract or supernatant from a cell or tissue culture). These candidate compounds may be novel or known. The concentration of the candidate compound to be added depends on the type of candidate compound. For example, for a low molecular weight compound, the concentration can be selected in the range of 1 nM to 10 mM. Incubation time may be in the range of 10 seconds to 72 hours.

The fluorescence intensity of the probe is then measured. The conditions for measuring fluorescence intensity in this embodiment may be the same as those defined in the first embodiment.

To determine whether the addition of a candidate compound has altered the fluorescence intensity of the probe, a reaction solution without the addition of the candidate compound may be analyzed in parallel for comparison, or the results of previously analyzed reaction solutions without the candidate compound may be used for comparison. In this method, if the fluorescence intensity of the probe in contact with the sample is significantly reduced compared to that of the probe in the reaction solution without the candidate compound, it can be determined that the candidate compound can inhibit the interaction between DPRs and LLPS-associated proteins/NPC family proteins, i.e., it can be determined that the candidate compound can normalize LLPS regulation, and thus, the candidate compound may be further evaluated as a promising therapeutic or prophylactic agent for neurodegenerative diseases.

A schematic of this method is shown in Figure 2. When the probe interacts with the LLPS-associated protein/NPC family protein, the probe fluoresces, but when the interaction is disrupted, the probe dissociates from the LLPS-associated protein/NPC family protein and the fluorescence intensity of the probe is reduced (turn-off response). Therefore, according to the method of the present embodiment, compounds that inhibit the interaction between DPRs and LLPS-associated proteins/NPC family proteins can be screened and thereby normalize LLPS regulation, based on the probe fluorescence intensity attenuation. Such compounds have potential as therapeutic or prophylactic agents for neurodegenerative diseases.

### EXAMPLE

An example is given below to further explain the present invention. Note that these examples do not in any way limit the scope of the present invention.

### <1. Materials and reagents>

### (1-1) Probes

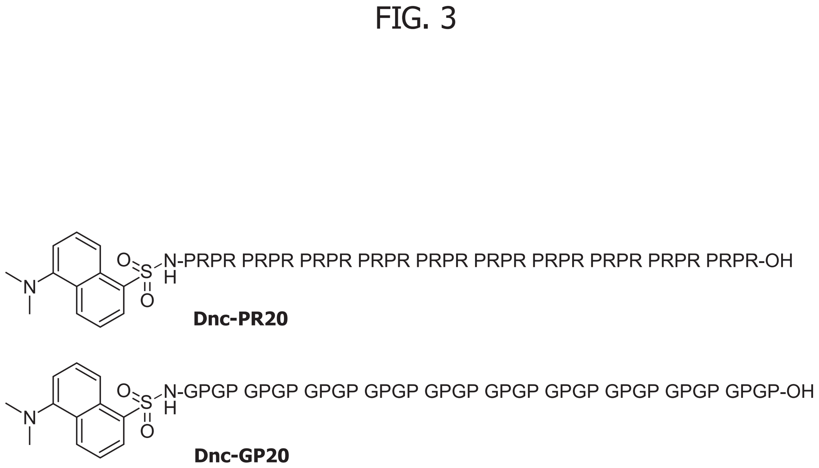

- Probes 1 to 7 (purity >95%) were synthesized by Biologica Co., Ltd., and were used without further purification. The structural formulae of probe 1 (sequence number 1) and probe 2 (sequence number 2) are shown in Figure 3, as representative examples.
- Probe 1: Dansyl-NH-(Pro-Arg)₂₀-COOH (sequence number 1) (hereinafter referred to as "Dnc-PR20")
- Probe 2: Dansyl-NH-(Gly-Pro)₂₀-COOH (sequence number 2) (hereinafter referred to as "Dnc-GP20")
- Probe 3: Dansyl-NH-(Pro-Arg)₁₀-COOH (sequence number 3) (hereinafter referred to as "Dnc-PR10")
- Probe 4: Dansyl-NH-(Gly-Pro)₁₀-COOH (sequence number 4) (hereinafter referred to as "Dnc-GP 10")
- Probe 5: Dansyl-NH-(Gly-Ala)₁₉-COOH (sequence number 5) (hereinafter referred to as "Dnc-GA10")
- Probe 6: Dansyl-NH-(Gly-Arg)₁₀-COOH (sequence number 6) (hereinafter referred to as "Dnc-GR10")
- Probe 7: Dansyl-NH-(Pro-Ala)₁₀-COOH (sequence number 7) (hereinafter referred to as "Dnc-PA10")

### (1-2) Proteins

Bovine milk-derived β-lactoglobulin, human serum-derived α1-antitrypsin, human serum-derived albumin, human serum-derived apotransferrin, and chicken egg white-derived lysozyme were purchased from Sigma-Aldrich. Human serum-derived immunoglobulin G was purchased from Equitech-Bio, Inc.

MBP-tagged human FUS (Cell, 173(3):693-705.e22 (2018); hereinafter "MBP-FUS"), human karyopherin β2 (Cell, 173(3):693-705.e22 (2018); hereafter referred to as "Kapβ2"), *C. elegans* importin β family protein (UniProt accession number: A0A131MBF9; hereafter referred to as "Imb-2"), human heteronuclear ribonucleoprotein protein A2 (Cell, 163(4):829-839 (2015); hereafter described as "hnRNPA2LC"), and human peptidyl prolyl cis-trans isomerase A (Cell, 163(4):829-839 (2015); hereafter referred to as "PPIA") were provided by Professor Tomohide Saio of the Institute for Advanced Enzyme Research, University of Tokushima. Human importin α1 (Cell, 173(3):693-705.e22 (2018); hereinafter described as "Impα"), human importin β1 (Cell, 173(3):693-705.e22 (2018); hereinafter referred to as "Impβ"), GST-tagged M9M peptide (Cell, 173(3):693-705.e22 (2018); hereafter referred to as "GST-M9M"), and human GTP-bound Ran (Q69L) (UniProt accession number: P62826; hereinafter referred to as "RanGTP") were provided by Assistant Professor Takuya Yoshizawa, Faculty of Life Science, Ritsumeikan University.

### (1-3) Reagents

ATP, Tris-HCl, dithiothreitol (DTT), polyamidoamine dendrimer (PAMAM), and 50-mer polylysine (poly K50) were purchased from Sigma-Aldrich. NaCl was purchased from Fujifilm Wako Pure Chemicals.

### <2. Kapβ2-induced fluorescence changes in probes>

A buffer solution (20 mM Tris-HCl (pH 7.5), 150 mM NaCl, and 2 mM DTT (final concentration)) was used to prepare the probe solutions (final concentration 300 nM) for probes 1 to 7 above. Each probe solution was added to a 384-well microplate (Corning, 3575) at 30 µL/well using an automated pipetting system (Andrew+, Andrew Alliance). After incubation at 35°C for 10 min, fluorescence intensity (fluorescence wavelength: 520 nm) and fluorescence spectrum (fluorescence wavelength: 400-700 nm) at an excitation wavelength of 340 nm were measured using a microplate reader (Cytation 5, BioTek). Then, various concentrations of Kapβ2 solution (Kapβ2/pure water) were added to the 384-well microplate at 30 µL/well using an automated pipetting system. After incubation at 35°C for 10 minutes, fluorescence intensity and fluorescence spectrum were measured under the same conditions as above.

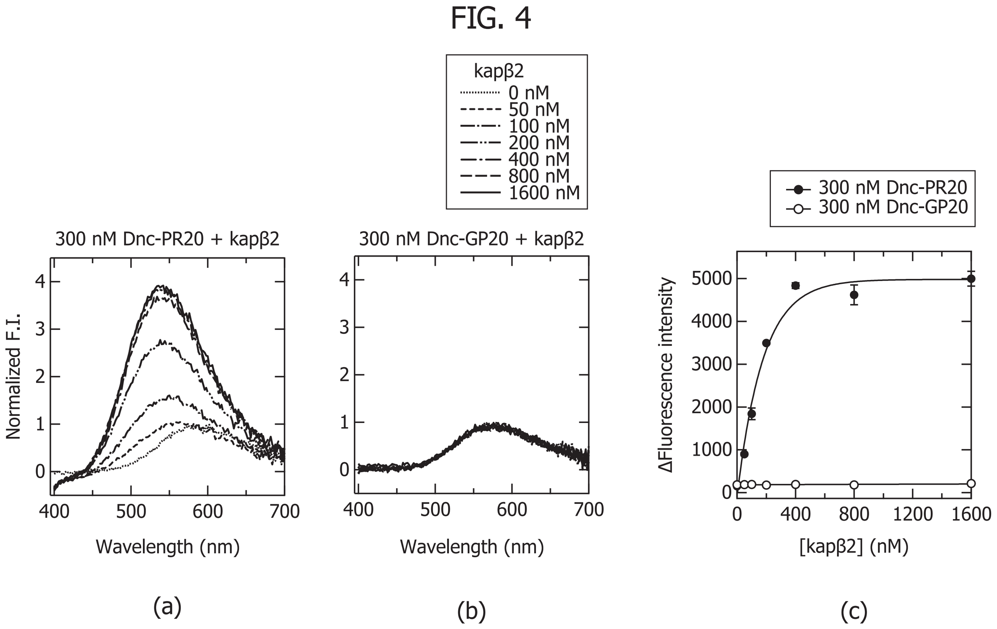

The results for probes 1 and 2 are shown in Figure 4. Figure 4(a) shows the fluorescence spectrum of Dnc-PR20 (probe 1), and Figure 4(b) shows the fluorescence spectrum of Dnc-GP20 (probe 2). The fluorescence intensity of Dnc-PR20 increased in a Kapβ2 concentration-dependent manner, with the fluorescence wavelength peak shifting from approximately 590 nm to 540 nm. The 540 nm fluorescence intensity of Dnc-PR20 at 1600 nM Kapβ2 increased approximately 6.1-fold compared to the fluorescence intensity at 0 nM Kapβ2. On the other hand, the fluorescence spectrum of Dnc-GP20 was little changed by the addition of Kapβ2. Figure 4(c) shows the change in the 520 nm fluorescence intensity for probes 1 and 2. The x-axis indicates the Kapβ2 concentration, and the y-axis indicates the mean + standard error of fluorescence intensity (n = 3). These results confirm that Dnc-PR20 binds to Kapβ2 but not Dnc-GP20.

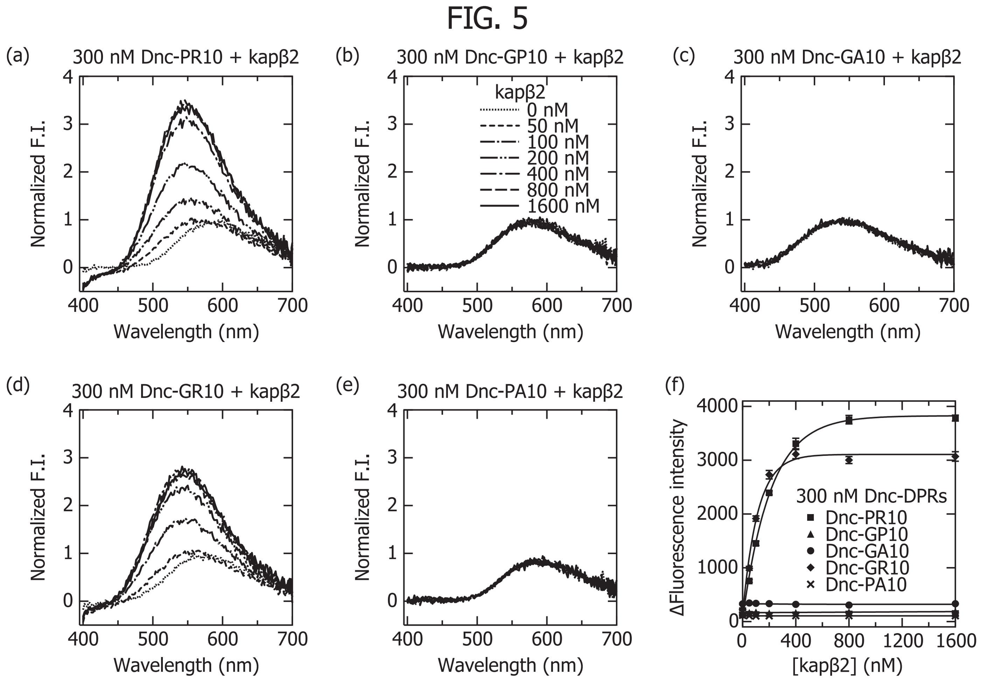

The results of probes 3 to 7 are shown in Figure 5. Figure 5(a) shows the fluorescence spectrum of Dnc-PR10 (probe 3), Figure 5(b) shows the fluorescence spectrum of Dnc-GP10 (probe 4), Figure 5(c) shows the fluorescence spectrum of Dnc-GA10 (probe 5), Figure 5(d) shows the fluorescence spectrum of Dnc-GR10 (probe 6), and Figure 5(e) shows the fluorescence spectrum of Dnc-PA10 (probe 7). Figure 5(f) shows the change in the 520 nm fluorescence intensity for probes 3 to 7. The x-axis indicates the Kapβ2 concentration, and the y-axis indicates the mean ± standard error of fluorescence intensity (n = 3). The fluorescence intensity of Dnc-PR10 and Dnc-GR10 increased in a Kapβ2 concentration-dependent manner, whereas the fluorescence intensity of Dnc-GP10, Dnc-GA10, and Dnc-PA10 were unchanged. These results confirm that Dnc-PR10 and Dnc-GR10 bind Kapβ2, but Dnc-GP10, Dnc-GA10, and Dnc-PA10 do not. These results were consistent with previous findings (Nat. Commun., 2021; 12(1):5301).

### <3. Fluorescence changes in probes due to FUS>

The same procedure as in Section 2 above was used, except that MBP-tagged FUS (MBP-FUS) solution (MBP-FUS/pure water) was used in place of the Kapβ2 solution, to measure the fluorescence intensity and fluorescence spectra of Dnc-PR20 (probe 1) and Dnc-GP20 (probe 2).

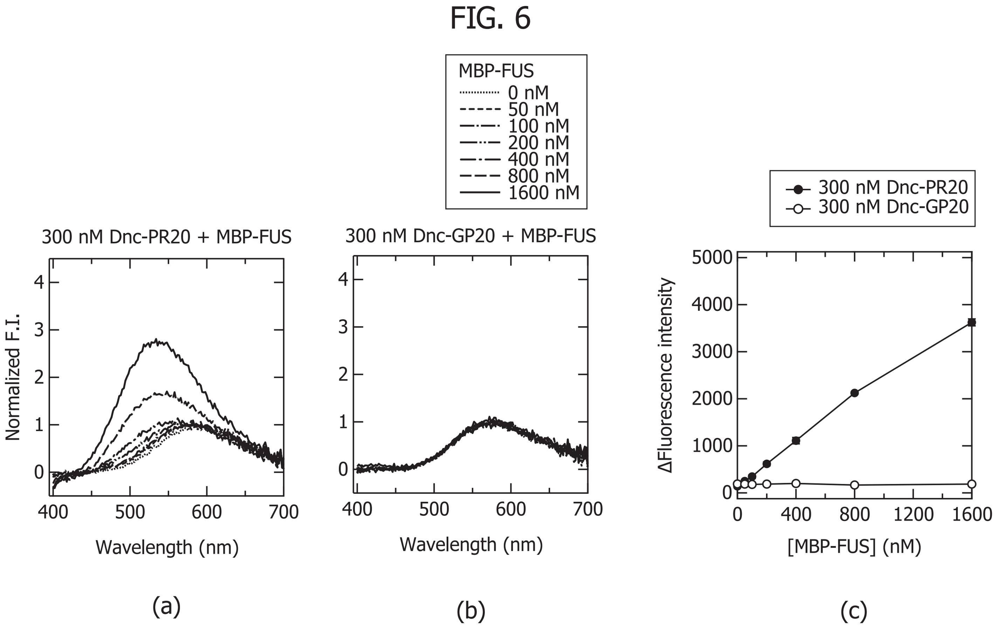

The results are shown in Figure 6. Figure 6(a) shows the fluorescence spectrum of Dnc-PR20 (probe 1), and Figure 6(b) shows the fluorescence spectrum of Dnc-GP20 (probe 2). The fluorescence intensity of Dnc-PR20 increased in an MBP-FUS concentration-dependent manner, with the fluorescence wavelength peak shifting from approximately 590 nm to 540 nm. The 540 nm fluorescence intensity of Dnc-PR20 at 1600 nM MBP-FUS increased approximately 4.6-fold compared to the fluorescence intensity at 0 nM MBP-FUS. On the other hand, the fluorescence spectrum of Dnc-GP20 hardly changed with the addition of MBP-FUS. Figure 6(c) shows the change in the 520 nm fluorescence intensity for probes 1 and 2. The x-axis indicates the MBP-FUS concentration, and the y-axis indicates the mean ± standard error of fluorescence intensity (n = 3). These results confirm that Dnc-PR20 binds to MBP-FUS but not Dnc-GP20.

### <4. Fluorescence change of probes by various proteins (1)>

The fluorescence intensity and fluorescence spectra of Dnc-PR20 (probe 1) and Dnc-GP20 (probe 2) were measured using the same procedure as described in Section 2 above, except that aqueous solutions (1600 nM) of bovine milk-derived β-lactoglobulin, human serum-derived α1-antitrypsin, human serum-derived albumin (HSA), human serum-derived apotransferrin, chicken egg white-derived lysozyme, or human serum-derived immunoglobulin G (IgG) were used in place of the Kapβ2 solution.

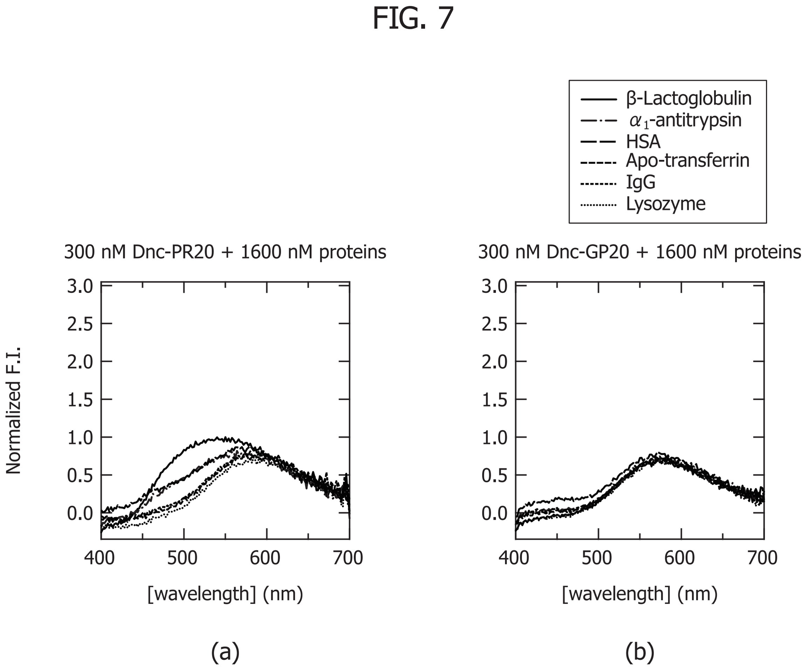

The results are shown in Figure 7. Figure 7(a) shows the fluorescence spectrum of Dnc-PR20 (probe 1), and Figure 7(b) shows the fluorescence spectrum of Dnc-GP20 (probe 2). The fluorescence spectrum of Dnc-PR20 showed that the addition of β-lactoglobulin shifted the fluorescence wavelength peak from approximately 590 nm to 540 nm and slightly increased the 540 nm fluorescence intensity. The fluorescence spectrum of Dnc-PR20 did not exhibit substantial changes with the addition of the other proteins. The fluorescence spectrum of Dnc-GP20 did not exhibit substantial changes with the addition of any of the proteins. These results indicate that Dnc-PR20 interacts slightly with β-lactoglobulin, but the interaction was much weaker than with Kapβ2 or MBP-FUS.

### <5. Fluorescence change of probes by various proteins (2)>

In addition to the proteins tested in Sections 2 to 4 above, aqueous solutions of proteins that may be relevant to the formation or control of liquid-liquid phase separation (LLPS) were used to measure the 520 nm fluorescence intensity of Dnc-PR20 (probe 1) and Dnc-GP20 (probe 2). The following proteins were used as proteins that may be associated with the formation or regulation of LLPS were Imb-2, hnRNPA2LC, PPIA, Impα, Impβ, GST-M9M, and RanGTP. The concentration of each protein solution was 1600 nM (except for Imb-2, which was 800 nM).

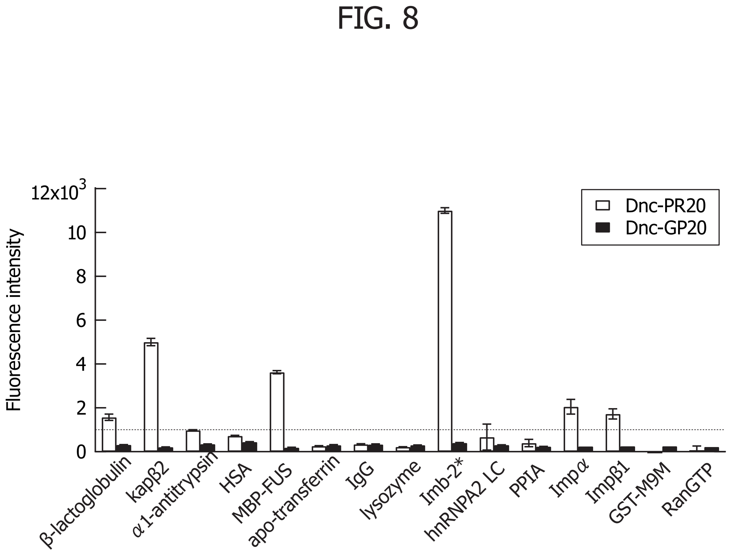

The results are shown in Figure 8. In addition to Kapβ2, MBP-FUS, and β-lactoglobulin confirmed in Sections 2 to 4 above, the addition of Imb-2, Impα, and Impβ also increased the 520 nm fluorescence of Dnc-PR20 above 1000. It has been previously reported that Kapβ2, importin α/β, and FUS bind to DPRs, and these results confirm that DPR-binding proteins can be detected by the increased fluorescence intensity of Dnc-PR20.

These results suggest that environment-sensitive fluorophore-DPR sequence-containing polypeptide probes can be used to screen for proteins that interact with DPRs.

### <6. Screening for DPRs-Kapβ2 binding inhibitors (1)>

A buffer solution (20 mM Tris-HCl (pH 7.5), 150 mM NaCl, and 2 mM DTT (final concentration)) was used to prepare a mixture of Dnc-PR20 (final concentration 300 nM) and Kapβ2 (final concentration 400 nM), and an automatic dispensing system (Andrew+, Andrew Alliance) was used to add 20 µL/well of each to a 384-well microplate (Corning, 3820). After incubation at 35°C for 10 min, the fluorescence intensity (fluorescence wavelength: 520 nm) and fluorescence spectrum (fluorescence wavelength: 400-700 nm) were measured at an excitation wavelength of 340 nm using a microplate reader (Cytation 5, BioTek). Then, 5 µL/well of various concentrations of additives (poly K50 or IgG/pure water) were added using an automated pipetting system, and after incubation at 35°C for 10 minutes, the fluorescence intensity and fluorescence spectrum were measured under the same conditions. As a control, the Dnc-PR20 solution without Kapβ2 was measured for fluorescence intensity and fluorescence spectrum in the same manner.

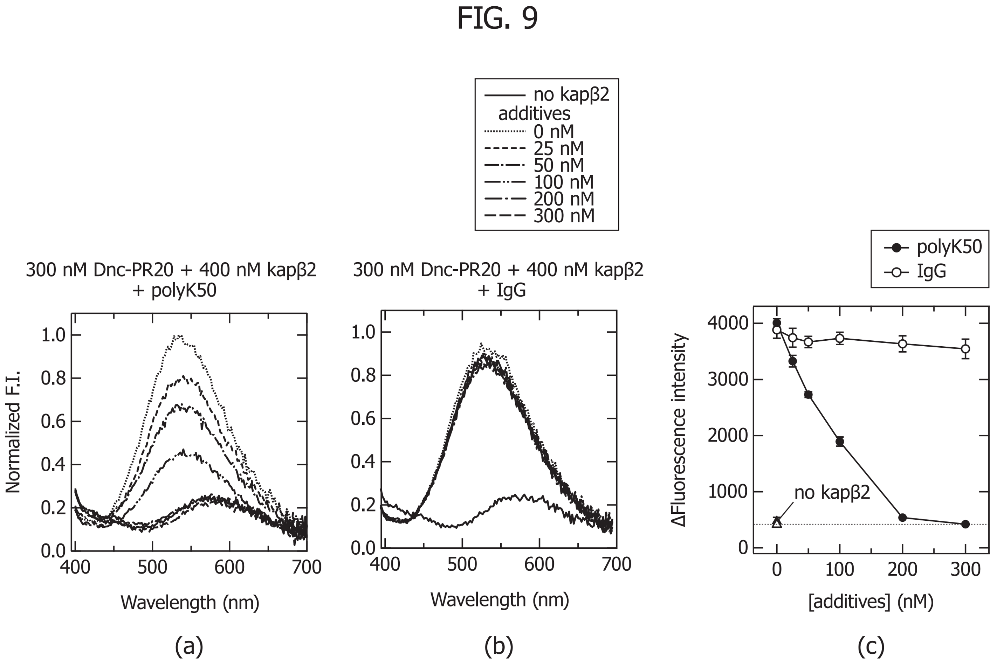

The results are shown in Figure 9. Figure 9(a) shows the fluorescence spectrum of the Dnc-PR20/Kapβ2 solution with poly K50 (0-300 nM), and Figure 9(b) shows the fluorescence spectrum of the DPRs/Kapβ2 solution with IgG (0-300 nM). When poly K50 was added to the Dnc-PR20/Kapβ2 solution, the fluorescence intensity of Dnc-PR20 decreased in a poly K50 concentration-dependent manner, and the addition of 200 nM of poly K50 resulted in a fluorescence intensity comparable to that of the control. On the other hand, the addition of IgG hardly changed the fluorescence intensity of Dnc-PR20. Figure 9(c) shows the change in the 520 nm fluorescence intensity. The x-axis indicates the additive concentration, and the y-axis indicates the mean ± standard error of fluorescence intensity (n = 3). These results indicate that poly K50 inhibited the interaction between Dnc-PR20 and Kapβ2. Without wishing to be bound by any particular theory, poly K50, like PR20, is cationic, but it is thought to interact more strongly with Kapβ2 because it has more charges per molecule than PR20.

### <7. DPRs-Kapβ2 binding inhibition for the screening of agents (2) >

In addition to poly K50 and IgG, the 520 nm fluorescence intensity of Dnc-PR20 was measured with PAMAM, GST-M9M, RanGTP, ATP, and HSA as additives, using the same procedure as in Section 6 above.

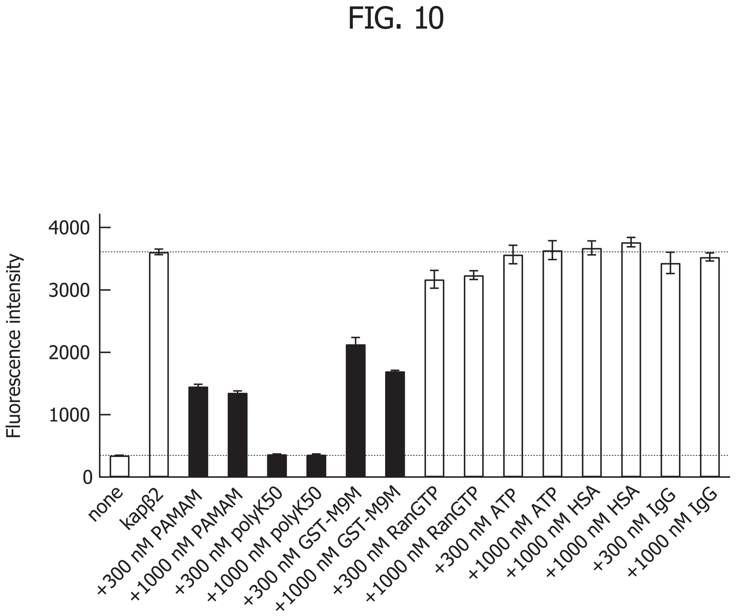

The results are shown in Figure 10. In the figure, "none" indicates the Dnc-PR20 solution without Kapβ2, and "Kapβ2" indicates the Dnc-PR20/Kapβ2 mixture. In addition to poly K50, the addition of PAMAM, which is a cationic synthetic dendrimer, and M9M peptide, which is a known Kapβ2 inhibitor, attenuated the 520 nm fluorescence intensity of Dnc-PR20. The fluorescence intensity of Dnc-PR20 was little changed by the other additives. These results indicate that compounds that inhibit the interaction of DPRs with Kapβ2 can be detected by Dnc-PR20 fluorescence intensity attenuation.

These results suggest that environmentally sensitive fluorophore-DPR sequence-comprising polypeptide probes can be used to screen for compounds that inhibit the interaction between DPRs and Kapβ2.

### <8. Screening for DPRs-Kapβ2 binding inhibitors (3)>

### (8-1) Screening of a protein-protein interaction inhibitor library

A demonstration experiment to screen for compounds that inhibit the interaction between DPRs and Kapβ2 was conducted using 47 protein-protein interaction (PPI) inhibitors selected from the PPI inhibitor library (TargetMol, L9400). A mixture of Dnc-PR20 (final concentration 300 nM) and Kapβ2 (final concentration 400 nM), prepared in the manner as for Section 6 above, was added to a 384-well microplate (Corning, 3820) using an automated pipetting system (ASSIST PLUS, Integra Biosciences) at 20 µL/well. After incubation at 35°C for 10 minutes, the fluorescence intensity (fluorescence wavelength of 520 nm) at an excitation wavelength of 340 nm was measured by a microplate reader (Synergy H1, Agilent Technologies). Then, using an automated pipetting system, 5 µL/well of various PPI inhibitors (compound Nos. 01 to 47, compound/25 vol% in DMSO) were added, incubated at 35°C for 10 minutes, and the fluorescence intensity was measured under the same conditions as above. As a control, the Dnc-PR20 solution without Kapβ2 was measured for fluorescence intensity.

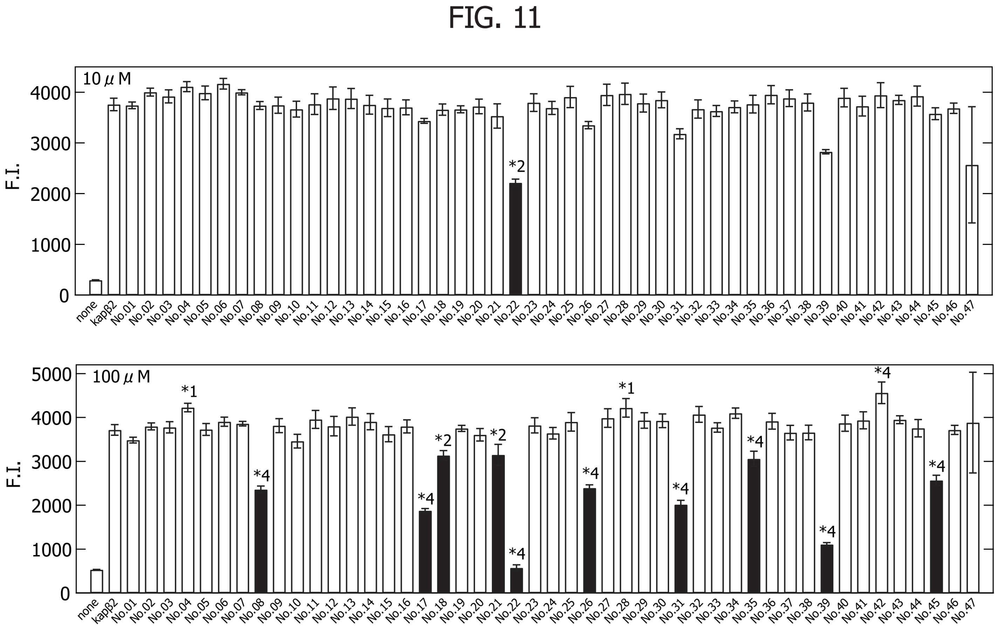

The results are shown in Figure 11. The upper and lower panels show the 520 nm fluorescence intensity (mean ± standard error) of the Dnc-PR20/Kapβ2 solutions with 10 µM and 100 µM PPI inhibitor, respectively. In the figure, "none" indicates the Dnc-PR20 solution without Kapβ2, and "Kapβ2" indicates the Dnc-PR20/Kapβ2 mixture. One compound at a concentration of 10 µM and 13 compounds at a concentration of 100 µM significantly altered the 520 nm fluorescence intensity of the solution (*1 P < 0.05, *2 P < 0.01, *4 P < 0.0001 for the Dnc-PR20/Kapβ2 mixture; Tukey post-hoc test with one-way ANOVA. Furthermore, 10 of the 13 compounds significantly attenuated the 520 nm fluorescence intensity of the solution (Figure 11, black bars).

### (8-2) DPRs-Kapβ2 binding inhibitory effect of compound No. 08

Next, the 520 nm fluorescence intensity of Dnc-PR20 was measured using the same procedure as in Section 8 above with compound No. 8, which represents the 10 compounds that significantly attenuated the 520 nm fluorescence intensity of the solution.

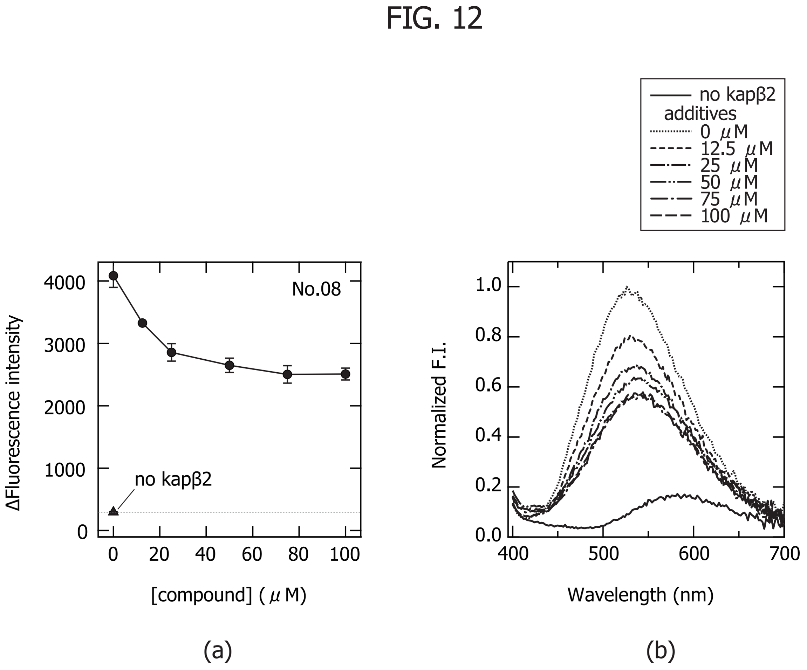

The results are shown in Figure 12. Figure 12(a) shows the change in the 520 nm fluorescence intensity of the Dnc-PR20/Kapβ2 solution with compound No. 8 (0-100 µM). The x-axis indicates the compound No. 8 concentration, and the y-axis indicates the mean ± standard error of fluorescence intensity (n = 3). Figure 12(b) shows the fluorescence spectra of the Dnc-PR20/Kapβ2 solution with compound No. 8 (0-100 µM). The fluorescence intensity of Dnc-PR20 decreased in a concentration-dependent manner for compound No. 8 up to 75 µM. These results indicate that compound No. 8 significantly inhibited the interaction between DPRs and Kapβ2, suggesting that compound No. 8 may be a promising DPRs-Kapβ2 binding inhibitor.

These results support that this screening method using the environment-sensitive fluorophore-DPR sequence-comprising polypeptide probe can select compounds that inhibit interaction between DPRs and Kapβ2.

## Claims

1. A method of screening for a protein associated with a neurodegenerative disease, comprising:
(1) contacting a probe comprising a polypeptide comprising a dipeptide repeat sequence and an environment-sensitive fluorophore covalently linked thereto with a sample containing a protein, wherein the dipeptide repeat sequence consists of a repeating unit selected from the group consisting of proline-arginine, glycine-arginine, proline-alanine, glycine-alanine, and glycine-proline; and
(2) measuring the fluorescence intensity of the probe.

2. The method as claimed in claim 1, wherein the dipeptide repeat sequence comprises 3 to 200 repeating units.

3. The method according to claim 1 or 2, wherein the environment-sensitive fluorophore is selected from the group consisting of fluorophores with a naphthalenesulfonic acid core, fluorophores with a benzofurazan core, fluorophores with a xanthene core, fluorophores with a pyrene core, and aggregation-induced emission (AIE) fluorophores.

4. The method of any one of claims 1 to 3, wherein the sample is a biological sample.

5. The method of any one of claims 1 to 4, wherein the neurodegenerative disease is amyotrophic lateral sclerosis or frontotemporal lobar degeneration.

6. A method of screening an agent for the treatment or prevention of a neurodegenerative disease, comprising:
(1) contacting, in the presence of a candidate compound, a probe comprising a polypeptide comprising a dipeptide repeat sequence and an environment-sensitive fluorophore covalently linked thereto, with a liquid-liquid phase separation-associated protein or a nuclear pore complex family protein, wherein the dipeptide repeat sequence consists of a repeating unit selected from the group consisting of proline-arginine, glycine-arginine, proline-alanine, glycine-alanine, and glycine-proline; and
(2) measuring the fluorescence intensity of the probe.

7. The method as claimed in claim 6, wherein the dipeptide repeat sequence comprises 3 to 200 repeating units.

8. The method according to claim 6 or 7, wherein the environment-sensitive fluorophore is selected from the group consisting of fluorophores with a naphthalenesulfonic acid core, fluorophores with a benzofurazan core, fluorophores with a xanthene core, fluorophores with a pyrene core, and aggregation-induced emission (AIE) fluorophores.

9. The method of any one of claims 6 to 8, wherein the liquid-liquid phase separation-associated protein is FUS.

10. The method of any one of claims 6 to 8, wherein the nuclear pore complex family protein is karyopherin β2.

11. The method of any one of claims 6 to 10, wherein the neurodegenerative disease is amyotrophic lateral sclerosis or frontotemporal lobar degeneration.
